# EUROPEAN PATENT SPECIFICATION

(11) **EP 1 183 377 B1**
(45) Date of publication and mention of the grant of the patent: **20.06.2007**
(21) Application number: 00936770.7
(22) Date of filing: 18.05.2000
(51) Int. Cl.: C12N 15/82

(54) **IMPROVED METHOD FOR AGROBACTERIUM MEDIATED TRANSFORMATION OF COTTON**
VERBESSERTE METHODE ZUR AGROBAKTERIEN-VERMITTELTEN TRANSFORMATION VON BAUMWOLLE
TECHNIQUE AMELIOREE DE TRANSFORMATION DE COTON INDUITE PAR AGROBACTERIUM

(30) Priority: 19.05.1999 US 314449
(43) Date of publication of application: 06.03.2002
(73) Proprietor: Bayer BioScience N.V., 9052 Gent (BE)
(72) Inventor: REYNAERTS, Arlette, B-9031 Drongen (BE); DE SONVILLE, Anne, B-9820 Merelbeke (BE)
(86) International application number: PCT/EP2000/004611
(87) International publication number: WO 2000/071733

(56) References cited:
- WO-A-89/05344
- WO-A-97/12512
- WO-A-97/43430
- WO-A-98/37212
- US-A- 4 954 442
- DATABASE CABA [Online] Accession number 2000:46062, 1997 XP002149079 & CHAIR H. ET AL.: "Agrobacterium mediated transformation of Sri Sumrong 60, a Thai cotton variety" KASETSART JOURNAL NATURAL SCIENCES, vol. 31, 1997, pages 149-156,
- VELUTHAMBI K ET AL: "OPINES STIMULATE INDUCTION OF THE VIR GENES OF THE AGROBACTERIUM-TUMEFACIENS TI PLASMID" JOURNAL OF BACTERIOLOGY, vol. 171, no. 7, 1989, pages 3696-3703, XP000946748 ISSN: 0021-9193 cited in the application
- FIROOZABADY ET AL: "Transformation ofcotton (Gossypium hirsutum L.) by Argrobacterium tumefaciens and regeneration of transgenic plants" PLANT MOLECULAR BIOLOGY,NL,NIJHOFF PUBLISHERS, DORDRECHT, vol. 10, 1987, pages 105-116, XP002094283 ISSN: 0167-4412 cited in the application
- HOSHINO, YOICHIRO ET AL: "Production of transgenic grapevine (Vitis vinifera L. cv. Koshusanjaku) plants by co-cultivation of embryogenic calli with Agrobacterium tumefaciens and selecting secondary embryos" PLANT BIOTECHNOL. (TOKYO) (1998), 15(1), 29-33 , XP000946719

## Description

This invention relates to the field of plant transformation, particularly to an improved method for *Agrobacterium* mediated transformation of cotton.

Cotton is an important crop, grown primarily for its lint, which provides much of the high quality fiber for the textile industry. Cotton seed is also a valuable commodity, as it provides a source for oil, meal and seed hulls.

About 90 % of cotton grown worldwide is *Gossypium hirsutum* L., whereas *Gossypium barbadense* accounts for the about 8 %.

Improvement of various traits such as resistance to pests, stress or disease, fiber quality, reduced gossypol content, has been obtained by conventional breeding techniques, and further improvement of these and other traits can be achieved using molecular and genetic techniques.

Several methods are available for the transformation of cotton including *Agrobacterium* mediated transformation of cotton explants, such as isolated cotyledons, isolated hypocotyls and isolated hypocotyl transition zones, as well as direct gene transfer by microprojectile bombardment of meristimatic cotton tissues.

Firoozabady et al. (1987, Plant Molecular Biology 10:105-116) described transformation of cotton cotyledon tissues by *Agrobacterium tumefaciens* and regeneration of transgenic plants.

Umbeck et al. (1987, Bio/Technology 5: 263-266) reported results on cotton transformation via *Agrobacterium* using hypocotyls as explants.

PCT publication ("WO") 89/05344, US patent ("US") 5, 244,802 and US 5,583,036 provide methods for regeneration of cotton by tissue and suspension culture starting with explants which are the hypocotyl, cotyledon or immature embryos. Also taught are method to transform cotton and improve cotton by selective growth.

WO 89/12102 and US 5,164,310 relate to a novel method for transforming and rapidly regenerating plant tissue. The method employs target tissues which are the shoot apices, thereby expanding the species range for transformation and reducing the risk of somaclonal variation.

WO 98/15622 provides a method for the *in vitro* regeneration of fertile *Gossypium* plants in which cells from the transition region tissue of seedlings is excised and cultured. A method for the production of transgenic *Gossypium* plants capable of transmitting a foreign gene to progeny is also described in which cells derived from the transition region tissue of seedlings are targeted for transformation.

WO 97/12512 provides a method for regenerating cotton plants from explant tissue which allows the generation of embryogenic callus from a cotton tissue explant, such as a hypocotyl, which is not cultivated on callus initiation media having exogenous plant hormones. The method can be utilized in the *Agrobacterium-mediated* transformation of cotton plants.

US 5,004,863 and US 5,159,135 and European patent publication "EP" 0 270 355 all disclose a method to achieve genetic transformation of cotton plants and lines, by subjecting immature cotton tissues such as hypocotyl segments in vitro to *Agrobacterium* mediated genetic transformation.

WO 97/43430 relates to a versatile method of rapidly regenerating cotton plants from explants of apical and/or nodal meristematic tissues which can be coupled with well known methods of transformation such as *Agrobacterium-*mediated transformation for the rapid production of genetically-engineered cotton varieties of agronomic importance.
WO 92/15675 and EP 0 531 506 describe a method for the particle-mediated transformation of cotton permitting the direct genetic engineering of elite cotton lines without the need for tissue culture or callus proliferation, involving excision of the embryonic axes from germinating seeds and blasting particles carrying foreign genes into the embryonic axes.

Finer and Mc Mullen (1990, Plant Cell Reports, 8: 586-589) described transformation of cotton via particle bombardment of embryogenic suspension cultures.

McCabe and Martinell, (1993, Bio/Technology 11: 596-598) have described transformation of elite cotton cultivars via particle bombardment of cotton meristematic tissue from excised embryonic axes.

Hansen et al. (1994, Proc. Natl. Acad. Sci. 91: 7603-7607) have described an *Agrobacterium* strain comprising a constitutive *vir*G mutant (viiGN54D) leading to enhanced transformation efficiencies of isolated cotton cotyledons.

Veluthambi et al. (1989, Journal of Bacteriology 171: 3696-3703) described a markedly increased transformation of cotton shoot tips by the simultaneous addition of acetosyringone and nopaline at the time of infection.

The cotton *Agrobacterium-mediated* transformation methods of the prior art suffer from the major drawback, as indicated in Murray et al. (1993, in Transgenic Plants Vol 2, Academic Press Inc. p153-168) that the time required to regenerate a transgenic plant from an explant which has been cocultivated with *Agrobacterium* cells is extremely long.

Moreover, an additional problem arises from the fact that all described *Agrobacterium* mediated transformation methods of cotton require the generation of embryogenic callus from the explant containing the transformed cells, as an intermediate step in the regeneration of transgenic cotton plants. Since not all transformed cells of the explant necessarily are competent for the initiation of embryogenic callus, a loss of regeneration of a number of potentially transgenic cells, which consequently results in a low transformation efficiency, is observed with these methods. An increase in starting numbers of putative transformed cotton explants to compensate for this loss, results in an huge increase of the amount of labor to be invested in obtaining regenerated transgenic cotton plants.

US patent 4,954,442 discloses that a mixture of opine (nopaline) and acetosyringone improves the : efficiency of Agrobacterium-mediated transformation of cotton shoot apices.

Hoshino et al., 1998 (Plant Biotechnol. 15(1) 29-33 describes the production of transgenic grapevine plants by co-cultivation of embryogenic calli with Agrobacterium tumefaciens and selecting secondary embryos. Transformation efficiency of the embryogenic calli evaluated by GUS histochemical assay was increased by the addition of acetosyringone to the co-culture medium.

WO98/3721 describes a process for integrating a DNA fragment into the genome of a cell of a monocotyledonous plant, the process comprising the steps of: 1) incubating, prior to contacting with the DNA fragment, a culture of untransformed monocotyledonous plant cells on a medium comprising a plant phenolic compound, for a period of time sufficient to stimulate cell division and enhance competence for integration of foreign DNA; and 2) contacting the untransformed cells with the DNA fragment under conditions in which the DNA fragment is taken up by the untransformed cells and is stably integrated in the genome of the untransformed cells, to generate transformed cells.

WO 89/05344 has suggested in general terms that embryogenic callus may be used as appropriate starting material for *Agrobacterium* mediated transformation of cotton callus. Nevertheless, during the past decade no reports were made of successful production of transgenic cotton plants using cocultivation of embryogenic callus with *Agrobacterium.*

To some extent, the problem has been alleviated by techniques in microprojectile bombardment of meristematic tissue which results in a larger number of transgenic cotton lines. Nonetheless, the generation of germline transformed cotton plants, which are capable of passing on the transgene to their progeny, is infrequent.

The art is thus deficient in providing an efficient method, both in term of required time and transformation efficiency, for the generation of transgenic cotton plants which are capable of transferring the foreign incorporated DNA to their progeny plants.

### Summary and objects of the invention

In view of the foregoing shortcomings associated with prior art cotton transformation processes as well as other disadvantages not specifically mentioned above, it should be apparent that there still exists a need in the art for a method of transforming cotton which achieves the heretofore elusive combination of a high transformation efficiency in a relatively short period of time. It is therefore a primary objective of the present invention to fulfill that need by providing a method for producing a transgenic cotton plant, preferably a fertile transgenic plant, by *Agrobacterium-*mediated transformation, comprising cocultivating *Agrobacterium* cells comprising a DNA fragment of interest operably linked to at least one T-DNA border with cotton embryogenic callus in the presence of a plant phenolic compound.

It is another object of the invention to provide a method for producing a transgenic cotton plant which eliminates the step of generating embryogenic callus from a cotton explant comprising the transformed cells, thereby both reducing the potential loss of transformed cotton lines from transformed plant cells which are not capable of generating embryogenic callus, and in this way enhancing transformation frequencies.

In a first aspect, the present invention relates to a method for producing a transgenic cotton plant comprising incubation of *Agrobacterium* cells comprising a DNA fragment of interest operably linked to at least one T-DNA border, with a plant phenolic compound prior to or during the co-cultivation of cotton embryogenic callus with the Agrobacterium cells.

In another aspect, the present invention relates to a method for producing a transgenic cotton plant, comprising:
i) cocultivating cotton embryogenic callus, preferably derived from a hypocotyl of a cotton seedling, with *Agrobacterium* cells, the *Agrobacterium* cells comprising a DNA fragment of interest operably linked to at least one T-DNA border, in the presence of a plant phenolic compound, preferably selected from the group consisting of acetosyringone, α-hydroxy-acetosyringone, sinapinic acid, syringic acid, ferulic acid, catechol, p-hydroxybenzoic acid, β-resorcylic acid, protocatechuic acid, pyrrogallic acid, gallic acid and vanillin, preferably present in a concentration range from about 50 µM to about 400 µM, for a time sufficient to generate embryogenic callus comprising a transformed cotton cell, preferably for about 3 to 4 days;
ii) regenerating a transgenic cotton plant from said transformed cell; and optionally
iii) crossing said transgenic cotton plant with another cotton plant

In another aspect, the invention relates to the use of a plant phenolic compound, preferably acetosyringone, for *Agrobacterium* mediated transformation of cotton embryogenic callus.

### Detailed description of the invention

The inventors have developed an improved and accelerated *Agrobacterium* mediated transformation method for the production of transgenic cotton plants, comprising the use of a plant phenolic compound, such as acetosyringone, during *Agrobacterium* mediated transformation of cotton embryogenic callus, preferably during the cocultivation stage of cotton embryogenic callus with *Agrobacterium* cells. This method on average leads to a time reduction in the period starting from cocultivation towards the time point at which the transgenic cotton plant is sufficiently developed to be transferred to soil, preferably under greenhouse conditions, of at least about 2 to 3 months.

Since the improved method of this invention comprises the use of embryogenic callus as starting tissue for the transformation, the step of generating embryogenic callus from the cotton explant comprising the transformed cells is eliminated. Consequently, the potential loss of transformed cotton lines from transformed plant cells which are not capable to generate embryogenic callus is reduced, and transformation frequencies are enhanced.

Moreover, embryogenic callus, once initiated, can be maintained straightforwardly (as long as somaclonal variation does not interfere with phenotype of regenerated plants) thus facilitating the management of the supply of starting material for transformation experiments.

Indeed, the inventors have surprisingly found that *Agrobacterium* mediated transformation of cotton embryogenic callus, necessitated the addition of a plant phenolic compound, such as but not limited to acetosyringone, preferably at the cocultivation stage. Nowhere in the prior art has it been suggested that the inclusion of such a plant phenolic compound, of all possible variables, would lead to successful *Agrobacterium* mediated transformation of cotton embryogenic callus.

In one embodiment, the invention relates to the use of a plant phenolic compound during the Agrobacferium-mediated transformation of cotton embryogenic callus.

"Plant phenolic compounds" or "plant phenolics" suitable for the invention are those substituted phenolic molecules which are capable to induce a positive chemotactic response, particularly those who are capable to induce increased *vir* gene expression in a Ti-plasmid containing *Agrobacterium* sp., particularly a Ti-plasmid containing *Agrobacterium tumefaciens.* Methods for measuring chemotactic response towards plant phenolic compounds have been described by Ashby et al. (1988, J. Bacteriol. 170: 4181-4187) and methods to measure induction of *vir* gene expression are also well known (Stachel et al., 1985 Nature 318: 624-629; Bolton et al. 1986, Science 232: 983-985).

Preferred plant phenolic compounds are those found in wound exudates of plant cells. One of the best known plant phenolic compounds is acetosyringone, which is present in a number of wounded and intact cells of various plants, albeit it in different concentrations. However, acetosyringone (3,5-dimethoxy-4-hydroxyacetophenone) is not the only plant phenolic which can induce the expression of *vir* genes. Other examples are α-hydroxy-acetosyringone, sinapinic acid (3,5 dimethoxy-4-hydroxycinnamic acid), syringic acid (4-hydroxy-3,5 dimethoxybenzoic acid), ferulic acid (4-hydroxy-3-methoxycinnamic acid), catechol (1,2-dihydroxybenzene), p-hydroxybenzoic acid (4-hydroxybenzoic acid), β-resorcylic acid (2,4 dihydroxybenzoic acid), protocatechuic acid (3,4-dihydroxybenzoic acid), pyrrogallic acid (2,3,4 - trihydroxybenzoic acid), gallic acid (3,4.5-trihydroxybenzoic acid) and vanillin (3-methoxy-4-hydroxybenzaldehyde), and these phenolic compounds are known or expected to be able to replace acetosyringone with similar results. As used herein, the mentioned molecules are also referred to as plant phenolic compounds.

Plant phenolic compounds can be used either alone or in combination with other plant phenolics. Certain compounds, such as osmoprotectants (e.g. L-proline or betaine), phytohormes, *(inter alia* NAA), opines, or sugars, are expected to act synergistically when added in combination with plant phenolic compounds.

In a preferred embodiment, the invention relates to a method for transforming a cotton plant, i.e. producing a transgenic cotton plant by introduction of a DNA fragment of interest into the genome of the cells of a cotton plant, wherein the method comprises :
a) cocultivating cotton embryogenic callus with *Agrobacterium* cells, said *Agrobacterium* cells comprising a DNA fragment of interest operably linked to at least one T-DNA border, in the presence of a plant phenolic compound, for a time sufficient to generate embryogenic callus comprising a transformed cotton cell; and
b) regenerating a transgenic cotton plant from said transformed cell.

The methods provided by the invention employ cotton embryogenic callus as starting material for the cocultivation with the *Agrobacterium* cells. As used herein "cotton embryogenic callus" or "embryogenic callus derived from a cotton explants" refers to compact or solid callus tissue, cultivated on solid media, derived from a cotton explant, which can be regenerated into an intact cotton plant through somatic embryogenesis. This definition of embryogenic cotton callus does not include embryogenic suspension cells, cultured in liquid medium.

Methods to induce embryogenic callus from a cotton explant have been disclosed in the art, e.g. in WO 97/12512.

It will be clear for the skilled artisan, that the source of the cotton explant from which embryogenic callus has been derived is only of limited importance for the method of the current invention. Embryogenic callus may be derived from any suitable cotton explant such as but not limited to hypocotyls, cotyledons, immature or mature embryos etc.

Preferably, the cotton embryogenic callus used in the transformation methods described herein, was obtained by incubation of cotton hypocotyl explants on hormone free callus induction medium as described in WO 97/12512.

"Cocultivation" is used herein to indicate the process of contacting between the embryogenic cotton callus and the Agrobacterium cells, prior to the addition of bacteriostatic or bacteriocidal compounds to inhibit or eliminate the Agrobacteria. The "cocultivation stage" of an Agrobacterium mediated transformation method thus refers to the time period between the initial contacting of the Agrobacteria with the plant tissue, until the time point where a bacteriostatic or bacteriocidal compound is added to the medium with the intention to remove or inhibit the Agrobacterium strains comprising the DNA fragment of interest.

"A DNA fragment of interest" is used herein to describe any DNA fragment which one would like to introduce into the genome of cells of a cotton plant, irrespective of whether it encodes a protein or polypeptide, RNA or not. Indeed, a DNA fragment may be introduced mainly for tagging purposes. The only prerequisite for the DNA fragment of interest is that it should be "operably linked to at least one T-DNA border".

As used herein, "a T-DNA border" encompasses a DNA fragment comprising an about 25 nucleotide long sequence capable of being recognized by the virulence gene products of an *Agrobacterium* strain, preferably an A. *tumefaciens* or *A. rhizogenes* strain, and sufficient for transfer of the operably linked DNA to eukaryotic cells, preferably plant cells, particularly sufficient for transfer and integration of the operably linked DNA into the genome of a eukaryotic cell, preferably a plant cell. It is clear that this definition encompasses all naturally occurring T-DNA borders from wild-type plasmids, as well as any functional derivative thereof, including chemically synthesized T-DNA borders.

Preferably, the DNA of interest is located between two T-DNA borders, preferably on T-DNA vector capable of forming a hybrid Ti-plasmid by single cross-over through a fragment of DNA homology between the T-DNA vector and the helper Ti-plasmid.

The use of a plant phenolic compound according to the invention increases the transformation efficiency of *Agrobacterium* mediated DNA transformation of cotton embryogenic callus, and it is expected that this effect is independent of the chromosomal background of the *Agrobacterium* host, the type of Ti-plasmid, helper-plasmid or T-DNA vector used.

Particularly preferred bacterial chromosomal backgrounds are provided by *A*. *tumefaciens* C58C1 (Van Larebeke et al., 1974, Nature 252: 169-170), A136 (Watson et al., 1975, J. Bacteriol 123: 255-264) or LBA4011 (Klapwijk et al., 1980. J. Bacteriol 141, 128-136).

in a preferred embodiment, the *Agrobacterium* strain used to transform the plant tissue precultured with the plant phenolic compound contains a LL-succinamopine type Ti-plasmid, preferably disarmed, such as pEHA101.

The method of the invention can also be used in combination with particular *Agrobacterium* strains, to further increase the transformation efficiency, such as *Agrobacterium* strains wherein the *vir* gene expression and/or induction thereof is altered due to the presence of mutant or chimeric *vir*A or *vir*G genes (e.g. Hansen et al., 1994, supra; Chen and Winans, 1991, J. Bacteriol. 173: 1139-1144; Scheeren-Groot et al., 1994, J. Bacteriol. 176: 6418-6246).

In another embodiment, *Agrobacterium* strains comprising an extra *virG* gene copies, particularly the so-called super *vir*G gene derived from pTiBo542, preferably linked to a multiple-copy plasmid, may be used to further increase the transformation efficiency.

The concentration of the plant phenolic compound in the cocultivation medium is also believed to have an effect on the transformation efficiency. However, within certain concentration ranges, the effect is minimal. The optimal concentration range of plant phenolic compounds in the cocultivation medium may vary depending on the species or variety from which the cotton embryogenic callus is derived, but it is expected that about 100 µM is a suitable concentration for many purposes. The optimal concentration may also depend on the nature of the specific plant phenolic compound used, particularly on its cell-division promoting strength.

It was found for instance that the optimal concentration for acetosyringone is approximately 100 µM, but concentrations as low as approximately 25 µM can be used to obtain a good effect on transformation efficiency. Likewise, it is expected that higher concentrations up to approximately 400 µM will yield similar effects.

Comparable concentrations apply to other plant phenolic compounds, and optimal concentrations can be established easily by experimentation in accordance with this invention.

Typical co-cultivation times to generate an cotton embryogenic callus comprising transformed cells, are about 3 to about 4 days, but it is expected that a cocultivation time of one day may be sufficient to generate at least some transformed cells. Further, it is expected that cocultivation times should not exceed about 7 days.

It is clear for the skilled artisan that in addition to inclusion of a plant phenolic compound during cocultivation of cotton embryogenic callus with *Agrobacterium* cells, the used *Agrobacterium* cells may also be preinduced by incubation with a plant phenolic compound for a time sufficient to induce the virulence genes of the *Agrobacterium* cells.

Moreover, it is expected that that preinduction of *Agrobacterium* cells with a plant phenolic compound for a time sufficient to induce the virulence genes of the *Agrobacterium* cells, might obviate the requirement for inclusion of a plant phenolic compound during the cocultivation stage.

Thus, in another preferred embodiment, the invention relates to a method for transforming a cotton plant, i.e. producing a transgenic cotton plant by introduction of a DNA fragment of interest into the genome of the cells of a cotton plant, wherein the method comprises :
a) incubating an *Agrobacterium* cell culture comprising a DNA fragment of interest operably linked to at least one T-DNA border, with a plant phenolic compound, for a time sufficient to induce virulence genes of said *Agrobacterium* cells;
b) cocultivating embryogenic cellus derived from a cotton explant with said *Agrobacterium* cells incubated with said plant phenolic compound, so as to generate embryogenic callus comprising a transformed cotton cell; and
c) regenerating a transgenic cotton plant from said transformed cell.

Methods to preinduce the virulence genes of *Agrobacterium* cells by incubation with a plant phenolic compounds, as well as methods to determine whether the virulence genes of an *Agrobacterium* strain are induced, are available in the art, e.g. as described by Stachel *et al.,* 1985 or Bolton *et al.,* 1986.

Optimized methods for induction of Agrobacteria with plant phenolic compounds, particularly acetosyringone, have been described e.g. by Vernade et al., (1988, J. of Bacteriology 170: 5822-5829) incorporated herein by reference.

The time of incubation of agrobacterial cells with a plant phenolic compound sufficient to induce the virulence genes may range from a few hours to about two days prior to cocultivation, but typically about 1 day incubation prior to cocultivation will be sufficient. Likewise, concentration of the plant phenolic compound may range from about 50 µM to about 400 µM, but typically about 100 µM will be a preferred concentration for the plant phenolic compound.

After cocultivation, the cotton embryogenic callus is preferably submitted to a selection regime to separate transformed cells from untransformed cells or to at least enrich for transformed cells.

To this end, the foreign DNA used in the method of this invention preferably also comprises a marker gene, the expression of which allows the separation of transformed cells from non-transformed cells. Such a marker gene generally encodes a protein which allows to phenotypically distinguish transformed cells from untransformed cells. In the case of a selectable marker gene, resistance to an antibiotic or other chemical compound that is normally toxic for the cells is generally provided to the cell by expression of that marker gene. In plants the selectable marker gene may thus also encode a protein that confers resistance to a herbicide, such as a herbicide comprising a glutamine synthetase inhibitor (e.g. phosphinothricin) as an active ingredient. An example of such genes are genes encoding phosphinothricin acetyl transferase such as the sfr or sfrv genes (EP 0 242 236; EP 0 242 246; De Block et al., 1987, EMBO J. 6: 2513-2518). However, it can also be that the distinguishable phenotype cannot be selected for, as is the case for marker genes normally referred to a screenable marker genes, including but not limited to, green fluorescent proteins (GFP) or β-glucuronidases (GUS) or β-lactamases well known in the art.

Moreover, using methods such as PCR-based DNA amplification methods, any DNA sequence, including part of the sequence of the DNA of interest, may be used as a tag for verification of the presence of the DNA fragment of interest.

Selection of transformed embryogenic callus cells is preferably performed on solid media, but can also be supplemented or replaced by a selection phase in liquid medium.

It goes without saying that during the selection and, if required, the regeneration phase, the growth of *Agrobacterium* has to be inhibited, preferably the *Agrobacterium* cells have to be eliminated. This can be achieved, as well known in the art, by inclusion into the media of an antibiotic, toxic to Agrobacteria, such as but not limited to cefotaxime.

Regeneration of the transformed embryogenic callus into transgenic cotton plant lines, can performed according to methods which are well within the skills of the art, such as but not limited to the regeneration methods described in WO 98/15622.

In a preferred embodiment, small embryo's are selected for germination and development into mature plants during the regeneration phase, preferably by growing the embryogenic callus in liquid medium on a gyratory shaker, and withholding for germination that fraction of the liquid suspension of embryogenic calli or somatic embryos which pass through a sieve, such as a tea-sieve.

A transgenic " cotton plant line" as used herein, consists of a group of transgenic cotton plants, originating from one transformed embryogenic callus piece, obtained during the regeneration process. In general, plants from one plant line are genetically identical, and originate from one transformation event, thus comprising the same transgenes integrated at the same genomic positions. However, individual plants from one plant line can originate from independent transformation events when using *Agrobacterium-mediated* DNA transfer, and may thus differ from one another. When transformation frequencies are expressed by the number of plant lines / 100 initial embryogenic callus pieces, it may be that the actual transformation frequencies (transformation events/ 100 initial callus pieces) are even higher.

The methods of the invention are particularly suited for the transformation of all cotton plants from which embryogenic callus can be derived (both Gossypium *hirsutum* and Gossypium *barbadense)* particularly for Coker 312, Coker310, Coker 5Acala SJ-5, GSC251 10, FiberMax 819 , Siokra 1-3, T25, GSA75, Acala SJ2, Acala SJ4, Acala SJ5. Acala SJ-C1, Acala B1644, Acala B1654-26. Acala B1654-43, Acala B3991. Acala GC356. Acala GC510, Acala GAM1, Acala C1, Acala Royale, Acala Maxxa, Acala Prema, Acala B638, Acala B1810, Acala B2724, Acala B4894, Acala B5002, non Acala "picker" Siokra, "stripper" variety FC2017, Coker 315, STONEVILLE 506, STONEVILLE 825, DP50, DP61, DP90, DP77, DES119, McN235, HBX87, HBX191, HBX107, FC 3027, CHEMBRED A1, CHEMBRED A2, CHEMBRED A3, CHEMBRED A4, CHEMBRED B1, CHEMBRED B2, CHEMBRED B3, CHEMBRED C1, CHEMBRED C2, CHEMBRED C3, CHEMBRED C4, PAYMASTER 145, HS26, HS46, SICALA, PIMA S6 and ORO BLANCO PIMA and plants with genotypes derived thereof.

The obtained transformed cotton plant can be used in a conventional breeding scheme to produce more transformed cotton plants with the same characteristics or to introduce the DNA fragment of interest into other varieties of the same or related cotton species, and methods further comprising such breeding activities are also included within the scope of the invention.

The following Examples describe the methods of the invention in detail. Unless stated otherwise in the Examples, all recombinant DNA techniques are carried out according to standard protocols as described in Sambrook et al. (1989) Molecular Cloning: A Laboratory Manual, Second Edition, Cold Spring Harbor Laboratory Press, NY and in Volumes 1 and 2 of Ausubel et al. (1994) Current Protocols in Molecular Biology, Current Protocols, USA. Standard materials and methods for plant molecular work are described in Plant Molecular Biology Labfax (1993) by R.D.D. Croy, jointly published by BIOS Scientific Publications Ltd (UK) and Blackwell Scientific Publications, UK.

### Examples

### Example 1: Media, plasmids and bacterial strains used in the Examples

### 1.1 Media

| Min A-glucose (MAG: *Agrobacterium* growth medium) | |
|---|---|
| K₂HPO₄.3H₂O | 13.8 g/l H₂O |
| KH₂PO₄ | 4.5 g/l |
| (NH₄)₂SO₄ | 1.0 g/l |
| sodium citrate | 0.5 g/l |
| MgSO₄.7H₂O | 0.4 g/l |
| thiamine | 5.0 g/l |
| glucose | 2.0 g/l |
| agar | 12.0 g/l |

| AB medium (*Agrobacterium* growth medium) | | |
|---|---|---|
| AB buffer (20x stock): | K₂HPO₄ | 60 g/l |
| | NaH₂PO₄.H₂O | 22.6 g/l |
| | | |
| AB salts (20x stock): | NH₄Cl | 20 g/l |
| | MgSO₄.7H₂O | 6 g/l |
| | KCI | 3 g/l |
| | CaCl₂.2H₂O | 0.26 g/l |
| | FeSO₄.7H₂O | 50 mg/l |

1 liter AB medium contains 50 ml AB buffer, 50 ml AB salts, 5 g/l glucose and
12 g/l agar if appropriate.

### Medium 100:

Murashige & Skoog salts+
30g/l glucose
10 mg/l thiamine
100 mg/l inositol
1 mg/l nicotinic acid
1 mg/l pyridoxine
0.5 g/l MES buffer
pH=5.8
Add 1.6g/l gelrite and 0.75 g/l MgCl₂.6H₂O whenever solid medium 100 is required

### Medium 104:

Murashige & Skoog salts+
30g/l glucose
10 mg/l thiamine
100 mg/l inositol
1 mg/l nicotinic acid
1 mg/l pyridoxine
1 g/l potassium nitrate
0.5 g/l MES buffer
pH=5.8
Add 2.0 g/l gelrite and 0.94 g/l MgCl₂.6H₂O whenever solid medium 104 is required

| Medium 700: | |
|---|---|
| Stewards Macro elements: | |
| KNO₃ | 0.506 g/l |
| NH₄NO₃ | 0.240 g/l |
| MgSO₄.7H₂O | 0.493 g/l |
| KH₂PO₄ | 0.027 g/l |
| CaCl₂.2H₂O | 0.176 g/l |

| Stewards Micro elements: | |
|---|---|
| Kl | 0.25 mg/l |
| MnSO₄.H₂O | 3.84 mg/l |
| H₃BO₃ | 1.85 mg/l |
| ZnSO₄.7H₂O | 2.59 mg/l |
| Na₂MoO₄.2H₂O | 0.22 mg/l |
| CuSO₄.5H₂O | 0.0075 mg/l |
| CoCl2.6H20 | 0.0071 mg/l |
| FeEDTA | 0.7 ml/I of a stock solution containing 0.54 g FeCl₃.6H₂O and 0.74 g Na₂EDTA in 100 ml H₂0 |
| Vitamins: | thiamine 1.5 mg/l |
| | pyridoxine 1.0 mg/l |
| | nicotinic acid 0.5 mg/l |
| sucrose: 20 g/l | |
| pH=6.8 | |

Solidify the medium with 1 g/l gelrite + 0.47 g/l MgCl₂.6H₂O and 2.25 g/l agar

### Medium 701:

Same composition as medium 700 but with 30 g/l glucose
solidify the medium with 20 g/l agar

### Medium 702:

Same composition as medium 700 but with 5 g/l sucrose
solidify the medium with 1.5 g/l gelrite + 0.71 g/l MgCl₂.6H₂O and 5 g/l agar.

### 1.2 Plasmids and bacterial strains

### Agrobacterium tumefaciens strain A3311

A3311 is A. *tumefaciens* strain C58C1Rif^{R} comprising the helper Ti-plasmid pEHA101 (Hood et al. 1986, J. of Bacteriology, 168: 1291-1301) and T-DNA vector pTHW136. Plasmid pTHW136 has been described in WO 98/31822.

### Agrobacterium tumefaciens strain A3784

A3784 is A. *tumefaciens* strain C58C1Rif^{R} comprising the helper Ti-plasmid pEHA101 (Hood *et al.* 1986) and T-DNA vector pTC0192. pTCO192 is similar to pGSV71 (WO 98/37212) and differs from the latter only in the presence of a region homologous to the nptl gene of pEHA101, located outside of the T-DNA borders.

### Agrobacterium tumefaciens strain A3724

A3724 is A. *tumefaciens* strain C58C1 RifR comprising the helper Ti-plasmid pEHA101 (Hood *et al.* 1986) and T-DNA vector pTSVH9901.
T-DNA vector pTSVH9901 is derived from the T-DNA cloning vector pGSV5 (described in WO 98/31822), comprising between its border sequences a chimeric plant expressible bar gene, under control of a CaMV35S promoter and followed by a 3' end formation signal from a nopaline synthase gene. T-DNA vector pTSVH9901 also contains between its border sequences a chimeric plant expressible Cry9C gene, wherein the CRY9C encoding open reading frame is inserted between a CaMV35S promoter and 3' end region, and wherein a cab22L leader has been inserted in the untranslated mRNA encoding region. The T-DNA vector further comprises a region, homologous to the nptl gene of pEHA101, located outside of the T-DNA borders.

### Agrobacterium tumefaciens strain A3727

A3311 is A. *tumefaciens* strain C58C1Rif^{R} comprising the helper Ti-plasmid pEHA101 (Hood *et al.* 1986) and T-DNA vector pTSHV0203.
T-DNA vector pTSHV0203 is based on the T-DNA cloning vector pGSV5 (WO 98/31822), comprising between its border sequences a chimeric plant expressible bar gene, under control of a CaMV35S promoter and followed by a 3' end formation signal from a nopaline synthase gene. T-DNA vector pTSHV0203 also contains between its border sequences a chimeric plant expressible Cry1Ab gene, wherein the CRY1Ab encoding open reading frame is inserted between a CaMV35S promoter and a 3' end region from the octopine synthase gene. The T-DNA further comprises a region homologous to the nptl gene of pEHA101, located outside of the T-DNA borders

### Example 2: Inclusion of a plant phenolic compound such as acetosyringone during cocultivation allows Agrobacterium mediated transformation of embryogenic cotton callus.

Embryogenic cotton callus was obtained in the following way:

Cotton seeds of variety Coker 312 were disinfected using a 12% sodium hypochloride solution, and germinated individually in tubes containing a solution of gelrite 2/g/l H₂O and 0.94 g/l MgCl₂.6H₂O. The tubes were incubated in the dark at 27-28°C. After 10 to 14 days, seedlings are collected, the hypocotyls of the seedlings were cut in segments of about 1 cm and incubated on medium 104 (without plant hormones) at 28°C under a regime of 16hr light and 8 hr dark. Every three weeks, the segments were transferred to fresh medium, until embryogenic callus was formed (usually after about 9 weeks). This callus was removed from the explants and cultured further on medium 104.

*Agrobacterium* strain A 3311 cells were grown on AB medium or MAG medium, collected and resuspended in M104 liquid medium, pH 5.2 (either with or without 100 µM acetosyringone (AS)) or MAG medium at a density of about 1 x 10⁹ CFU/ml. Small embryogenic cotton callus clumps were collected, washed in liquid medium 104 at pH 5.2, and immersed in the *Agrobacterium* suspension for about 20 minutes. The infected embryogenic calli were than transferred to solid medium 104, pH 5.2, supplemented with either 100 µM AS, 0.5 g/l casamino acids, 100 µM AS and 0.5 g/l casamino acids, or not supplemented at all, and incubated for cocultivation during 4 days in the dark at about 24 °C.

After the cocultivation phase, the callus clumps were transferred to selective medium 104 supplemented with 50 mg/l kanamycin and 500mg/l Claforan.

At different time intervals after the cocultivation, samples of embryogenic callus were collected and subjected to a β-glucuronidase assay, using a chromogenic substrate as described by Jefferson et al. (1987; Plant Mol. Biol. Rep. 5: 387-405). The results of these tests are summarized in table I, and are expressed as the percentage of calli expressing GUS-positive spots.

As a control, experiments were included using tobacco leaf discs cocultivated with A3311, using mock-inoculated tobacco leaf discs or using mock-inoculated embryogenic cotton callus.

It is clear from the results that inclusion of acetosyringone is required during *Agrobacterium* mediated transformation of embryogenic callus, both for transient expression and after prolonged cultivation of the transformed calli.

**Table 1. Overview of results of the effect of different parameters on Agrobacterium mediated transformation of cotton embryogenic callus (cotton EC).**

| **Tissue** | ***Agrobacte rium* strain** | ***Agrobacterium* resuspension medium** | **Cocultivation medium** | **4 days** | **11 days** | **4 weeks** | **6 weeks** | **8 weeks** |
|---|---|---|---|---|---|---|---|---|
| Cotton EC | none | - | M104 | 0 | 0 | 0 | 0 | Dead |
| Cotton EC | none | - | M104 + 100 µM AS | 0 | 0 | 0 | 0 | Dead |
| Cotton EC | none | - | M 104 + 0.5 g/l cas. Acids | 0 | 0 | 0 | 0 | Dead |
| Cotton EC | none | - | M104 + 100 µM AS and 0.5 g/l cas. acids | 0 | 0 | 0 | 0 | Dead |
| Cotton EC | A3311 | MAG | M 104 | 0 | 0 | 0 | 0 | Dead |
| Cotton EC | A3311 | MAG | M 104 + 100 µM AS | 90 | 90 | 50 | 50 | 90 |
| Cotton EC | A3311 | MAG | M 104 + 0.5 g/l cas. Acids | 0 | 0 | 0 | 0 | Dead |
| Cotton EC | A3311 | MAG | M 104 + 100 µM AS and 0.5 g/l cas. acids | 95 | 90 | 50 | 70 | 70 |
| Cotton EC | A3311 | M 104 | M 104 | 0 | 0 | 0 | 0 | Dead |
| Cotton EC | A3311 | | M104 + 100 µM AS | 5 | 50 | 5 | 15 | 20 |
| Cotton EC | A331 | | M 104 + 0.5 g/l cas. Acids | 0 | 0 | 0 | 0 | Dead |
| Cotton EC | A3311 | | M 104 + 100 µM AS and 0.5 g/l cas. acids | 5 | 20 | 30 | 10 | 60 |
| Cotton EC | A3311 | M104+100 µM AS | M104 | 0 | 0 | 0 | 0 | Dead |
| Cotton EC | A3311 | M104+100µM AS | M104+100µM AS | 10 | 70 | 30 | 0 | 90 |
| cotton EC | A3311 | M104+100 µM AS | M104+0.5 g/l cas. Acids | 0 | 0 | 0 | 0 | Dead |
| cotton EC | A3311 | M104+100 µM AS | M104+100 µM AS and 0.5 g/l cas. acids | 10 | 50 | 5 | 70 | 80 |
| cotton EC | none | - | M 104 | 0 | 0 | 0 | 0 | Dead |
| cotton EC | none | - | M 104 + 100 µM AS | 0 | 0 | 0 | 0 | Dead |
| cotton EC | none | - | M 104 + 0.5 g/l cas. Acids | 0 | 0 | 0 | 0 | Dead |
| cotton EC | none | - | M 104 + 100 µM AS and 0.5 g/l cas. acids | 0 | 0 | 0 | 0 | Dead |
| cotton EC | A3311 | MAG | M 104 | 0 | 5 | 0 | 0 | Dead |
| cotton EC | A3311 | MAG | M 104 + 100 µM AS | 90 | 90 | 50 | 90 | 80 |
| cotton EC | A3311 | MAG | M 104 + 0.5 g/l cas. Acids | 0 | 0 | 0 | 0 | Dead |
| cotton EC | A3311 | MAG | M 104 + 100 µM AS and 0.5 g/l cas. acids | 90 | 90 | 50 | 50 | 60 |
| cotton EC | A3311 | M 104 | M104 | 0 | 0 | 0 | 0 | Dead |
| cotton EC | A3311 | M 104 | M104+100µM AS | 50 | 90 | 50 | 25 | 20 |
| cotton EC | A3311 | M 104 | M 104 + 0.5 g/l cas. Acids | 0 | 0 | 0 | 0 | Dead |
| cotton EC | A3311 | M104 | M104+100µM AS and 0.5 g/l cas. acids | 30 | 70 | 30 | 90 | 50 |
| cotton EC | A3311 | M 104 + 100 µM AS | M104 | 0 | 0 | 0 | 0 | Dead |
| cotton EC | A3311 | M104 + 100 µM AS | M104 + 100 µM AS | 30 | 90 | 90 | 80 | 50 |
| cotton EC | A3311 | M104 + 100 µM AS | M 104 + 0.5 g/l cas. Acids | 0 | 0 | 0 | 0 | Dead |
| cotton EC | A3311 | M104 + 100 µM AS | M104 + 100 µM AS and 0.5 g/l cas. acids | 30 | 70 | 30 | 50 | 20 |

### Example 3: Agrobacterium mediated transformation of cotton using embryogenic callus.

Cotton embryogenic callus (EC) was generated as described in Example 2. Clumps of embryogenic callus were collected and washed in liquid medium 104 pH 5.2

*Agrobacterium* strains A3784, A3724 and A3727 were grown for 3 to 4 days on AB medium pH 7.0, collected and resuspended at a density of approximately 1 to 5 x 109 cells/ml in liquid medium 104, pH 5.2 containing 100 µM acetosyringone.

Embryogenic callus clumps were immersed in the *Agrobacterium* suspension for maximum about 20 minutes, and transferred for cocultivation during 4 days in the dark at 24°C on solid medium 104, p H5.2 supplemented with 100 µM acetosyringone.

After this cocultivation, the pieces of callus were transferred to a selective medium 104, pH 5.8, without acetosyringone or plant hormones, supplemented with 500 mg/l Claforan® (to inhibit the development of the *Agrobacterium* cells) and 5 mg/l phosphinotricin (PPT) (to select specifically for growth of transgenic calli).

Healthy looking pieces of embryogenic callus, actively growing, were transferred every 3 weeks to fresh selective medium 104.

Part of the callus (about 200mg) was resuspended in 20 ml liquid medium 100, supplemented with 5 mg/l PPT in a 100 ml flask on a rotary shaker (100-120 rpm)

After two weeks culture in the liquid medium, large clumps were removed by passing the suspension through a tea-sieve. The fraction passed-through fraction of embryogenic suspension was left to settle for about 20 min, after which spent medium was removed and replaced by fresh medium 100. 2 ml of this suspension of small embryo's were plated on solid medium 104, supplemented with 5 mg/l PPT and incubated at 28°C.

After about 2 weeks, developing embryos with a size between 4 and 10 mm were transferred to medium 701 and further incubated in the dark at 28 °C . Smaller embryos were transferred to medium 104 overlaid with sterile filter paper (with or without 5 mg/L PPT) to allow increase in size, sufficient for transfer to medium 701.

After about 2 weeks, the embryos were transferred to medium 702 (with or without selective pressure) for germination, and incubated at 28 °C, 16 hr light /8 hr dark.

Well developed plantlets (which may require 2 to 3 transfers on medium 702) were transferred to small containers containing M700. When these plantlets have developed two to three true leaves they were transferred to bigger containers with medium 700.

When the plants were about 10 cm in height, they were transferred to soil under greenhouse conditions.

Molecular analyses (Phosphinotricin acetylation assay, Southern hybridization, and ELISA to detect cry gene expression if appropriate) were performed on the callus level.

The results of these experiments are summarized in Table 2.
Simultaneous *Agrobacterium* mediated transformation experiments of cotton cotyledons according to the methods known in the art, were performed for comparison.

**Table 2. Comparison of the results of Agrobacterium mediated transformation experiments using cotton cotyledons or embryogenic callus as starting material.**

| Exp. Nr | Cotton variety | Agro strain | Tissue | Selected lines | Embryogenic lines | PAT positive | Elisa | Copy number | Plant status | Time* (weeks) |
|---|---|---|---|---|---|---|---|---|---|---|
| 1 | Coker 312 | A 3784 | Cotyledon | 108/246 | 0/108 | NA | NA | NA | No regeneration | 21 |
| 2 | Coker 312 | A3784 | EC | 13/25 | 13/13 | 6/6 | NA | 1 copy: 1 | Plantlets of about 2 cm | 28 |
| | | | | | | | | low copy: 3 | | |
| | | | | | | | | multiple copy: 9 | | |
| 3 | Fibermax 819 | A3784 | EC | 98/125 | 98/98 | 15/15 | NA | In progress | Plantlets <2cm | 18 |
| | | | | | | | | | | |
| 4 | Coker 312 | A3724 | cotyledon | 190/237 | 47/190 | ND | 2/13 | | Plantlets <2cm | 32 |
| 5 | Coker 312 | A3724 | EC | 66/275 | 66/66 | 22/22 | 39/63 | 1 copy: 14 | 16 plants >10cm | 31 |
| | | | | | | | | low copy: 9 | | |
| | | | | | | | | multiple copy: 15 | | |
| | | | | | | | | | | |
| 7 | Coker 312 | A3727 | cotyledon | 94/97 | 9/94 | ND | ND | ND | No regeneration | 32 |
| 8 | Coker 312 | A3727 | EC | 84/460 | 84/84 | 39/42 | 33/76 | 1 copy: 3 | Plantlets of 2 cm | 30 |
| | | | | | | | | low copy: 8 | | |
| | | | | | | | | multiple copy: 12 | | |

| | | | | | | | | | | |
|---|---|---|---|---|---|---|---|---|---|---|
| NA: Not appropriate; ND Not determined. * time (expressed in weeks) between the start of the experiment and the determination of the "Plant status". | | | | | | | | | | |

## Claims

1. A method for producing a transgenic cotton plant, said method comprising:
a) cocultivating solid cotton embryogenic callus cultivated on solid media with *Agrobacterium* cells, said *Agrobacterium* cells comprising a DNA fragment of interest operably linked to at least one T-DNA border, in the presence of a plant phenolic compound capable of inducing increased *vir* gene expression in said *Agrobacterium* cells, for a time sufficient to generate embryogenic callus comprising a transformed cotton cell; and
b) regenerating a transgenic cotton plant from said transformed cell.

2. The method of claim 1, wherein said plant phenolic compound is present in a concentration range from about 50 µM to about 400 µM.

3. The method of claim 1, wherein said *Agrobacterium* cells are cocultivated in the presence of said plant phenolic compound for a time period of about 3 to 4 days.

4. The method of claim 1, wherein said embryogenic callus is derived from a hypocotyl of a cotton seedling.

5. The method of claim 1, wherein said plant phenolic compound is selected from the group consisting of acetosyringone, α-hydroxy-acetosyringone, sinapinic acid, syringic acid, ferulic acid, catechol, p-hydroxybenzoic acid, β-resorcylic acid, protocatechuic acid, pyrrogallic acid, gallic acid and vanillin.

6. The method of claim 5, wherein said plant phenolic compound is acetosyringone.

7. The method of claim 1, wherein said *Agrobacterium* cells are *Agrobacterium tumefaciens* strain C58C1 (pEHA101), further comprising a DNA fragment of interest.

8. The method of claim 1, further comprising the step of crossing said transgenic cotton plant with another cotton plant.

## Patentansprüche

1. Verfahren zur Herstellung einer transgenen Baumwollpflanze, wobei das Verfahren folgendes umfaßt:
a) Kokultivieren von festem, auf festem Medium kultiviertem embryogenen Baumwollkallus mit *Agrobacterium*-Zellen, wobei die *Agrobacterium-*Zellen ein interessierendes DNA-Fragment in operativer Verknüpfung mit mindestens einer T-DNA-Border enthalten, in Gegenwart einer pflanzlichen phenolischen Verbindung, die eine verstärkte vir-Genexpression in den *Agrobacterium*-Zellen zu bewirken vermag, und zwar über eine Zeitdauer, die ausreichend ist, um embryogenen Kallus, der eine transformierte Baumwollzelle enthält, zu erzeugen; und
b) Regenerieren einer transgenen Baumwollpflanze aus der transformierten Zelle.

2. Verfahren nach Anspruch 1, wobei die pflanzliche phenolische Verbindung in einem Konzentrationsbereich von ungefähr 50 µM bis etwa 400 µM vorliegt.

3. Verfahren nach Anspruch 1, wobei die *Agrobacterium-*Zellen über eine Zeitdauer von ungefähr drei bis vier Tagen in Gegenwart der pflanzlichen phenolischen Verbindung kokultiviert werden.

4. Verfahren nach Anspruch 1, wobei der embryogene Kallus von einem Hypokotyl einer Baumwoll-Keimpflanze stammt.

5. Verfahren nach Anspruch 1, wobei die pflanzliche phenolische Verbindung aus der Gruppe Acetosyringon, α-Hydroxyacetosyringon, Sinapinsäure, Syringasäure, Ferulasäure, Catechin, p-Hydroxybenzoesäure, β-Resorcylsäure, Protocatechusäure, Pyrogallussäure, Gallussäure und Vanillin stammt.

6. Verfahren nach Anspruch 5, wobei es sich bei der pflanzlichen phenolischen Verbindung um Acetosyringon handelt.

7. Verfahren nach Anspruch 1, wobei es sich bei den *Agrobacterium*-Zellen und dem *Agrobacterium-tumefaciens*-Stamm C58C1(pEHA101) handelt, der weiterhin ein interessierendes DNA-Fragment enthält.

8. Verfahren nach Anspruch 1, das weiterhin den Schritt Kreuzen der transgenen Baumwollpflanze mit einer anderen Baumwollpflanze umfaßt.

## Revendications

1. Procédé de production d'un cotonnier transgénique, ledit procédé comprenant :
a) la co-culture d'un cal embryogène solide de coton cultivé sur un milieu solide avec des cellules *d'Agrobacterium,* lesdites cellules *d'Agrobacterium* comprenant un fragment d'ADN d'intérêt lié de manière opérationnelle à au moins une bordure d'ADN-T, en présence d'un composé phénolique végétal susceptible d'induire une expression accrue du gène *vir* dans lesdites cellules *d'Agrobacterium,* pendant un temps suffisant pour générer un cal embryogène comprenant une cellule de coton transformée ; et
b) la régénération d'un cotonnier transgénique à partir de ladite cellule transformée.

2. Procédé selon la revendication 1, **caractérisé en ce que** ledit composé phénolique végétal est présent dans une plage de concentration comprise entre environ 50 µM et environ 400 µM.

3. Procédé selon la revendication 1, **caractérisé en ce que** lesdites cellules *d'Agrobacterium* sont co-cultivées en présence dudit composé phénolique végétal pendant une durée d'environ 3 à 4 jours.

4. Procédé selon la revendication 1, **caractérisé en ce que** ledit cal embryogène est issu d'un hypocotyle d'une plantule de coton.

5. Procédé selon la revendication 1, **caractérisé en ce que** ledit composé phénolique végétal est choisi dans le groupe constitué de l'acétosyringone, de l'α-hydroxy-acétosyringone, de l'acide sinapinique, de l'acide syringique, de l'acide férulique, du pyrocatéchol, de l'acide p-hydroxybenzoïque, de l'acide β-résorcylique, de l'acide protocatéchique, de l'acide pyrrogallique, de l'acide gallique et de la vanilline.

6. Procédé selon la revendication 5, **caractérisé en ce que** ledit composé phénolique végétal est l'acétosyringone.

7. Procédé selon la revendication 1, **caractérisé en ce que** lesdites cellules *d'Agrobacterium* sont la souche d'*Agrobacterium tumefaciens* C58C1 (pEHA101), comprenant en outre un fragment d'ADN d'intérêt.

8. Procédé selon la revendication 1, comprenant en outre l'étape de croisement dudit cotonnier transgénique avec un autre cotonnier.
